# FASCICULE DE BREVET EUROPEEN

(11) **EP 0 788 333 B1**
(45) Date de publication et mention de la délivrance du brevet: **05.01.2000**
(21) Numéro de dépôt: 95936610.5
(22) Date de dépôt: 25.10.1995
(51) Int. Cl.: A61F 2/38

(54) **PROTHESE D'ARTICULATION DU GENOU**
KNIEGELENKPROTHESE
KNEE JOINT PROSTHESIS

(30) Priorité: 26.10.1994 FR 9413054
(43) Date de publication de la demande: 13.08.1997
(73) Titulaire: Aurex, 3200 Vichy (FR); Recmed, 6250 Mougins (FR); Erami, 69006 Lyon (FR); Dambreville, Alain, 29000 Quimper (FR); Demesy, Michel, 68200 Mulhouse (FR); Ginefri, Jean-Paul, 21000 Dijon (FR); Hummer, Jacques, 54000 Nancy (FR); Meynet, Jean-claude, 72000 Le Mans (FR); Ruyssen, Sylvain, 32000 Auch (FR)
(72) Inventeur: DAMBREVILLE, Alain, F-29000 Quimper (FR); DEMESY, Michel, F-68200 Mulhouse (FR); GINEFRI, Jean-Paul, F-21000 Dijon (FR); HUMMER, Jacques, F-54000 Nancy (FR); MEYNET, Jean-Claude, F-72000 Le Mans (FR); RUYSSEN, Sylvain, F-32000 Auch (FR); CORNIC, Michel, 478, Chemin de Bel-Air F-06250 Mougins (FR); GACON, Gérard, F-69006 Lyon (FR); ROUSSEAU, Jacques-Marie, F-03270 Saint-Yorre (FR)
(74) Mandataire: Maureau, Philippe
(86) Numéro de dépôt international: FR9501416
(87) Numéro de publication internationale: WO9613232

(56) Documents cités:
- EP-A- 0 498 586
- DE-A- 4 031 520
- FR-A- 2 663 536

## Description

La présente invention concerne une prothèse d'articulation du genou.

Une prothèse du genou se compose classiquement :
- d'un élément fémoral, comprenant une tige médullaire ou des plots pour son ancrage au fémur et des surfaces convexes arrondies reproduisant les condyles fémoraux,
- d'un élément tibial, comprenant une tige médullaire ou des plots pour son ancrage au tibia et une extrémité supérieure plane, formant un plateau horizontal d'appui, et
- d'un plateau intermédiaire, comprenant des surfaces supérieures concaves, reproduisant les ménisques, recevant lesdites surfaces convexes arrondies de l'élément fémoral avec possibilité de glissement, et comprenant une face inférieure plane venant en appui contre la face supérieure de l'élément tibial.

Le plateau intermédiaire est généralement en polyéthylène de haute densité. Ce matériau favorise le glissement des surfaces en contact mais présente en revanche l'inconvénient important d'avoir une résistance à l'usure relativement faible, cette usure pouvant être importante compte tenu des contraintes répétées et s'exerçant plus ou moins en porte-à-faux que subit la prothèse lorsqu'elle est en charge.

Il existe des prothèses dans lesquelles ce plateau intermédiaire est fixé sur l'extrémité supérieure de l'élément tibial.

Un tel montage fixe s'avère toutefois ne pas permettre une reproduction parfaitement fidèle du mouvement naturel de l'articulation. Il peut en résulter une usure relativement rapide du plateau.

Pour remédier à ces inconvénients, il est connu de prévoir un plateau intermédiaire mobile par rapport à l'élément tibial dans un plan horizontal.

Dans certaines prothèses, le plateau intermédiaire peut pivoter autour d'un axe médian vertical, notamment par engagement d'un pion taisant saillie de sa face inférieure dans un alésage de forme correspondante aménagé dans l'élément tibial.

Dans d'autres prothèses, le plateau intermédiaire est monté coulissant dans un plan antéro-postérieur, notamment par engagement d'une ou plusieurs glissières aménagées dans sa face inférieure sur une ou plusieurs nervures de guidage en queue d'aronde que comporte l'élément tibial.

Dans d'autres prothèses encore, le plateau intermédiaire est mobile tant en pivotement autour d'un axe vertical qu'en coulissement dans le plan antéro-postérieur.

On connaît également une prothèse dont le plateau intermédiaire présente une face inférieure convexe et comprend une rainure antéro-postérieure médiane, et dont l'élément tibial présente une face supérieure concave, complémentaire de la face inférieure du plateau intermédiaire, et comprend un téton en forme de tronc de cône se trouvant en arrière du creux maximum de cette face supérieure, ce creux étant lui-même décalé sur l'avant de l'élément tibial. Ces faces convexes et concaves sont destinées à glisser l'une contre l'autre et ce téton est destiné à être engagé dans la rainure du plateau, en pouvant pivoter et coulisser à travers elle pour permettre le pivotement et le coulissement du plateau par rapport à l'élément tibial.

Avec l'ensemble des prothèses à plateau mobile existantes, on constate que la reproduction fidèle du mouvement naturel de l'articulation n'est pas parfaitement obtenue et qu'il subsiste une usure notable du plateau, tant sous l'effet des contraintes répétées résultant de la charge exercée sur la prothèse que sous l'effet des frottements existant au niveau des moyens de guidage du plateau en pivotement et/ou en coulissement. Cette usure peut affecter la fiabilité de ces prothèses.

En particulier, les arêtes plus ou moins vives que comprennent les moyens de guidage en pivotement et/ou en coulissement du plateau intermédiaire peuvent à la longue entamer le polyéthylène dont le plateau est constitué.

Il peut en résulter soit une réduction de la mobilité du plateau, soit un jeu de celui-ci par rapport à l'élément tibial, qui, dans un cas comme dans l'autre, accélèrent l'usure.

En outre, le matériau de scellement des éléments fémoral et tibial subit également des contraintes répétées, pouvant, à la longue, conduire à un descellement.

La présente invention vise à remédier simultanément à ces inconvénients, les plus fréquemment rencontrés dans des prothèses de genou, en fournissant une prothèse qui reproduise non seulement le mouvement naturel de l'articulation de la manière la plus fidèle possible mais qui permette également d'avoir une usure du plateau restant limitée.

Cette prothèse est du type possédant :
- d'une part, un plateau intermédiaire mobile en pivotement autour d'un axe vertical et en coulissement dans un plan antéro-postérieur, qui présente une face inférieure convexe ayant la forme d'une portion de calotte sphérique et qui comprend une rainure antéro-postérieure médiane, et,
- d'autre part, un élément tibial, qui présente une face supérieure concave ayant la forme d'une portion de calotte sphérique de même centre que celle de la face inférieure du plateau intermédiaire, et qui comprend un pion de guidage faisant saillie de cette face supérieure, destiné à être engagé dans ladite rainure en pouvant pivoter et coulisser à travers elle.

Dans la prothèse selon l'invention, en combinaison avec les caractéristiques indiquées ci-dessus :
- le creux maximum de la face supérieure concave de l'élément tibial est situé sensiblement en avant du centre géométrique de cette face supérieure ;
- le pion de guidage est situé sensiblement au niveau de ce creux maximum et comprend un corps de section transversale circulaire et une tête de plus grande largeur que le corps, également de section transversale circulaire ; et
- la rainure aménagée dans le plateau intermédiaire a une section transversale correspondant sensiblement au profil du pion de guidage, c'est-à-dire qu'elle peut recevoir ce pion avec possibilité de pivotement et de coulissement mais avec une possibilité réduite de jeu vertical.

Ainsi, dans la prothèse selon l'invention, le plateau est mobile en pivotement et en coulissement antéro-postérieur sans possibilité véritable de mouvement dans un plan vertical, ces mouvements de pivotement et de coulissement se produisant autour d'un point situé sensiblement en avant du centre géométrique de la face supérieure de l'élément tibial, et au niveau du creux maximum de cette face supérieure.

Cette prothèse s'avère reproduire fidèlement le mouvement naturel de l'articulation et n'engendrer qu'une usure faible du plateau intermédiaire.

En particulier, lesdites surfaces concaves et convexes précitées permettent le pivotement et le coulissement du plateau intermédiaire tout en assurant la stabilisation de celui-ci par rapport à l'élément tibial, notamment lorsque l'articulation est en extension. Cette stabilisation permet de limiter les efforts en porte-à-faux s'exerçant entre le plateau et le pion de guidage lorsque les charges subies par la prothèse sont importantes.

L'usure au niveau de la rainure reste limitée et n'affecte pas à la longue la mobilité du plateau. De plus, aucun jeu néfaste n'existe et n'est créé à l'usage entre le plateau et l'élément tibial.

De préférence, la tête du pion présente une forme sensiblement sphérique. Ainsi, il n'existe pas d'arête vive au niveau des surfaces de contact du pion avec le plateau, et ces surfaces sont relativement importantes. Il en résulte que le pion ne risque pas d'entamer le polyéthylène du plateau à la longue et que l'usure du plateau reste faible.

De préférence, la face supérieure du plateau intermédiaire, et donc les surfaces concaves qu'elle comprend pour recevoir les surfaces convexes arrondies de l'élément fémoral avec possibilité de glissement, est inclinée en direction de l'arrière du plateau, ce qui s'avère favoriser le déplacement de ce dernier selon un mouvement proche du mouvement anatomique et contribuer à limiter l'usure.

Avantageusement, le plateau d'appui que forme l'extrémité supérieure de l'élément tibial pour recevoir ledit plateau intermédiaire présente une épaisseur sensiblement constante, c'est-à-dire comprend une face inférieure convexe ayant la forme d'une portion de calotte sphérique de même centre que celle de la face supérieure de ce plateau d'appui.

Ainsi, ledit plateau d'appui présente des bords périphériques plus minces que ceux des plateaux d'appui classiques, de sorte qu'il est possible d'implanter cet élément tibial en opérant une résection de la corticale moins importante qu'avec les prothèses à plateau tibial ayant une face inférieure plane. Cette limitation de la résection osseuse est moins traumatisante pour le patient et favorise l'ostéo-intégration ultérieure de l'élément tibial. En outre, l'élément tibial selon l'invention est mieux stabilisé par rapport à l'os qu'un élément tibial classique.

La prothèse selon l'invention est destinée à être implantée sans conservation du ligament croisé antérieur mais, en principe, avec conservation du ligament croisé postérieur.

Lorsque le ligament croisé postérieur n'est pas trop détérioré et peut donc être conservé, la rainure antéro-postérieure précitée débouche sur la face latérale postérieure du plateau intermédiaire, ce qui facilite le montage, par coulissement, de ce plateau sur l'élément tibial.

Lorsque le ligament croisé postérieur ne peut être conservé, la rainure est fermée au niveau postérieur du plateau par une paroi transversale formant une butée limitant le mouvement du plateau vers l'avant. Dans ce cas, le plateau comprend, sensiblement en partie centrale de la rainure, deux encoches permettant le passage de la tête du pion en vue de son introduction dans la rainure.

Pour sa bonne compréhension, l'invention est à nouveau décrite ci-dessous en référence au dessin schématique annexé représentant, à titre d'exemples non limitatifs, l'élément tibial et deux formes de réalisation du plateau intermédiaire de la prothèse de genou qu'elle concerne.
La figure 1 est une vue en perspective de cet élément tibial et du plateau intermédiaire selon une première forme de réalisation, avant montage ;
la figure 2 est une vue en coupe de cet élément tibial et de ce plateau intermédiaire, en coupe selon un plan antéro-postérieur sensiblement médian, et, en silhouette, de l'élément fémoral que comprend également la prothèse ; et
la figure 3 est une vue en perspective similaire à la figure 1 de l'élément tibial et du plateau intermédiaire selon une deuxième forme de réalisation, avant montage.

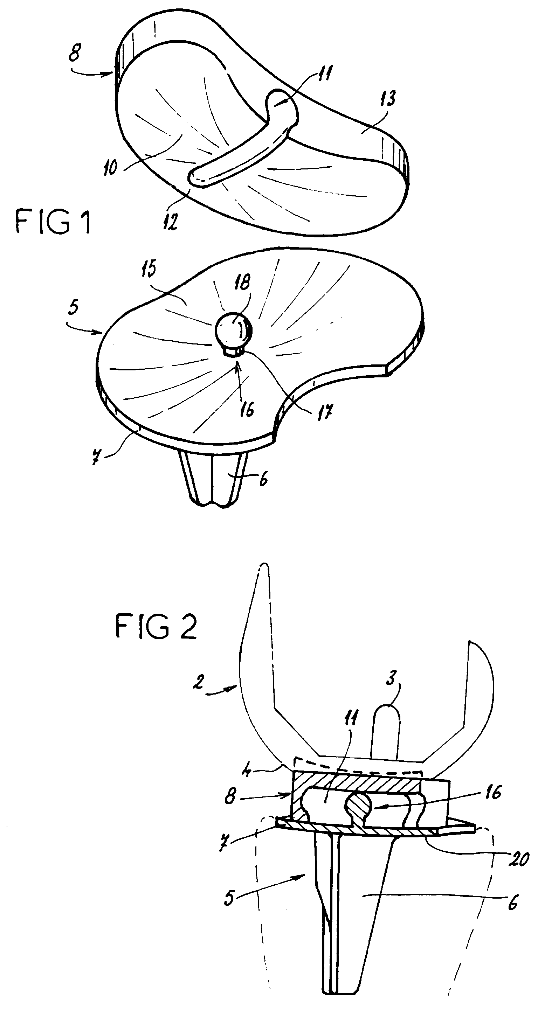

La figure 2 représente une prothèse d'articulation du genou se composant classiquement :
- d'un élément fémoral 2 comprenant des plots 3 pour son ancrage au fémur et des surfaces convexes arrondies 4 reproduisant les condyles fémoraux,
- d'un élément tibial 5, comprenant une tige médullaire 6 pour son ancrage au tibia et une extrémité supérieure plane, formant un plateau d'appui 7, d'orientation générale sensiblement horizontale et
- d'un plateau intermédiaire 8, comprenant des surfaces supérieures concaves (non visibles), reproduisant les ménisques, recevant les surfaces convexes arrondies 4 de l'élément fémoral 2 avec possibilité de glissement, et comprenant une face inférieure venant en appui contre la face supérieure du plateau d'appui 7.

Les éléments fémoral 2 et tibial 5 sont réalisés en métal, tandis que le plateau intermédiaire 8 est réalisé en polyéthylène de haute densité.

Comme le montrent plus particulièrement les figures 1 et 2, le plateau intermédiaire 8 présente une face inférieure 10 ayant la forme d'une portion de calotte sphérique et comprend une rainure antéro-postérieure médiane 11. Cette rainure 11 présente une largeur moindre au niveau de la face 10 et un fond de forme arrondie en section transversale. Elle est limitée antérieurement par une paroi transversale 12 et débouche sur la face latérale postérieure 13 du plateau intermédiaire 8.

L'élément tibial 5 présente une face supérieure 15 concave ayant la forme d'une portion de calotte sphérique de même centre que celle de la face inférieure 10 du plateau intermédiaire 8. Comme le montre plus particulièrement la figure 2, le creux maximum de cette face supérieure 15 est situé sensiblement en avant du centre géométrique de cette même face 15, à environ 5 millimètres de celui-ci.

L'élément tibial 5 comprend également un pion de guidage 16 faisant saillie de la face supérieure 15. Ce pion 16 est situé sensiblement au niveau du creux maximum de la face 15 et comprend un corps 17 de section transversale circulaire et une tête 18 de plus grande largeur que le corps, cette tête présentant une forme sensiblement sphérique.

Comme cela apparaît sur les figures 1 et 2, la rainure 11 a une section transversale correspondant sensiblement au profil du pion de guidage 16, c'est-à-dire qu'elle peut recevoir la tête 18 de ce pion au niveau de son fond et le corps 17 au niveau de son ouverture dans la paroi 10, avec possibilité de pivotement et de coulissement de ce pion dans la rainure mais avec une possibilité réduite de jeu vertical.

En outre, la face supérieure du plateau intermédiaire 8, et donc les surfaces concaves qu'elle comprend pour recevoir avec possibilité de glissement les surfaces convexes arrondies 4 de l'élément fémoral, est, comme le montre la figure 2, inclinée en direction de l'arrière du plateau 8.

Ainsi, dans la prothèse représentée aux figures 1 et 2, le plateau 8 est mobile en pivotement et en coulissement antéro-postérieur sans possibilité véritable de mouvement dans un plan vertical, ces mouvements de pivotement et de coulissement se produisant autour du pion 16, c'est-à-dire autour d'un point situé sensiblement en avant du centre géométrique de la face supérieure de l'élément tibial 5 et au niveau du creux maximum de cette face.

Cette prothèse s'avère reproduire fidèlement le mouvement naturel de l'articulation et n'engendrer qu'une usure faible du plateau intermédiaire.

L'inclinaison de la face supérieure du plateau intermédiaire 8 s'avère favoriser le déplacement de ce dernier selon un mouvement proche du mouvement anatomique et contribuer à limiter l'usure.

La prothèse représentée aux figures 1 et 2 est destinée à être implantée sans conservation du ligament croisé antérieur mais avec conservation du ligament croisé postérieur.

Grâce au fait que la rainure antéro-postérieure 11 débouche dans la face latérale postérieure 13 du plateau 8, le montage de ce plateau 8 sur l'élément tibial 5 est réalisé par coulissement de ce plateau 8 sur cet élément 5, donc de manière facile.

Par ailleurs, il apparaît sur les figures 1 et 2 que le plateau d'appui 7 présente une épaisseur sensiblement constante, c'est-à-dire comprend une face inférieure convexe 20 ayant la forme d'une portion de calotte sphérique de même centre que celle de la face supérieure 15 de ce plateau 7.

Ainsi, le plateau 7 présente des bords périphériques plus minces que ceux des plateaux d'appui classiques, dont la face inférieure est plane, de sorte qu'il est possible d'implanter cet élément tibial en opérant une résection de la corticale moins importante qu'avec un élément tibial classique à face inférieure plane. Cette limitation de la résection osseuse est moins traumatisante pour le patient et favorise l'ostéo-intégration ultérieure de l'élément tibial. En outre, l'élément tibial 5 est mieux stabilisé par rapport à l'os qu'un élément tibial classique.

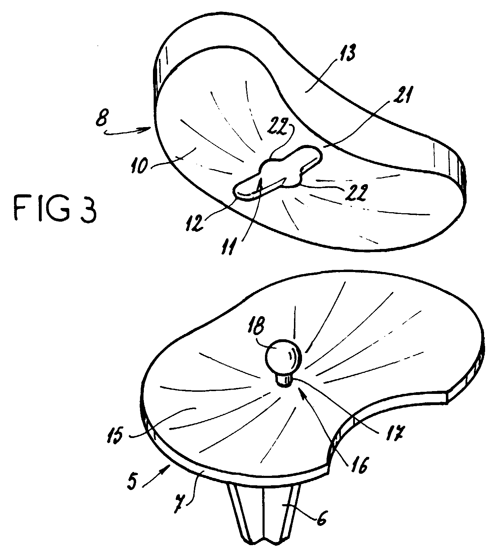

La figure 3 représente un élément tibial identique à celui qui vient d'être décrit et un plateau intermédiaire selon une deuxième forme de réalisation. Par souci de simplification, les éléments déjà décrits ci-dessus en référence aux figures 1 et 2 qui se retrouvent dans cette deuxième forme de réalisation sont désignés par les mêmes références numériques.

Selon cette deuxième forme de réalisation du plateau intermédiaire 8, la rainure 11 est fermée au niveau postérieur du plateau 8 par une paroi transversale 21, similaire à la paroi 12 précitée, formant une butée limitant le mouvement du plateau 8 vers l'avant. Pour permettre le passage de la tête 18 du pion 16 en vue de son introduction dans la rainure 11, le plateau 8 comprend, sensiblement en partie centrale de la rainure 11, deux encoches 22 permettant ledit passage.

Ce plateau intermédiaire 8 est utilisé lorsque le ligament croisé postérieur est trop détérioré pour être conservé.

## Revendications

1. Prothèse d'articulation du genou, possédant :
- d'une part, un plateau intermédiaire (8) mobile en pivotement autour d'un axe vertical et en coulissement dans un plan antéro-postérieur, qui présente une face inférieure (10) convexe ayant la forme d'une portion de calotte sphérique et qui comprend une rainure (11) antéro-postérieure médiane, et,
- d'autre part, un élément tibial (5), qui présente une face supérieure (15) concave ayant la forme d'une portion de calotte sphérique de même centre que celle de la face inférieure du plateau intermédiaire, et qui comprend un pion de guidage (16) faisant saillie de cette face supérieure (15), destiné à être engagé dans ladite rainure (11) en pouvant pivoter et coulisser à travers elle,
prothèse caractérisée en ce que
- le creux maximum de la face supérieure concave (15) de l'élément tibial (5) est situé sensiblement en avant du centre géométrique de cette face supérieure ;
- le pion de guidage (16) est situé sensiblement au niveau de ce creux maximum et comprend un corps (17) de section transversale circulaire et une tête (18) de plus grande largeur que le corps (17), également de section transversale circulaire ; et
- la rainure (11) aménagée dans le plateau intermédiaire (8) a une section transversale correspondant sensiblement au profil du pion de guidage (16), c'est-à-dire qu'elle peut recevoir ce pion (16) avec possibilité de pivotement et de coulissement mais avec une possibilité réduite de jeu vertical.

2. Prothèse selon la revendication 1, caractérisée en ce que la tête (18) du pion (16) présente une forme sensiblement sphérique.

3. Prothèse selon la revendication 1 ou la revendication 2, caractérisée en ce que la face supérieure du plateau intermédiaire (8), et donc les surfaces concaves qu'elle comprend pour recevoir les surfaces convexes arrondies (4) de l'élément fémoral (2) avec possibilité de glissement, est inclinée en direction de l'arrière du plateau (8).

4. Prothèse selon l'une des revendications 1 à 3, caractérisée en ce que le plateau d'appui (7) que forme l'extrémité supérieure de l'élément tibial (5) pour recevoir ledit plateau intermédiaire (8) présente une épaisseur sensiblement constante, c'est-à-dire comprend une face inférieure (20) convexe ayant la forme d'une portion de calotte sphérique de même centre que celle de la face supérieure de ce plateau d'appui (7).

5. Prothèse selon l'une des revendications 1 à 4, caractérisée en ce que la rainure antéro-postérieure (11) débouche sur la face latérale postérieure du plateau intermédiaire (8).

6. Prothèse selon l'une des revendications 1 à 4, caractérisée en ce que la rainure (11) est fermée au niveau postérieur du plateau (8) par une paroi transversale (21) formant une butée limitant le mouvement du plateau vers l'avant, le plateau (8) comprenant, sensiblement en partie centrale de la rainure (11), deux encoches (22) permettant le passage de la tête (18) du pion (16) en vue de son introduction dans la rainure (11).

## Claims

1. A knee joint prosthesis, of the type comprising
- firstly, an intermediate plate (8) which can move by pivoting about a vertical axis and by sliding in an anteroposterior plane, which comprises a convex lower face (10) having the shape of a portion of a spherical cap, and which comprises a median anteroposterior groove (11),
- secondly, a tibial element (5) which comprises a concave upper face (15) having the shape of a portion of a spherical cap with the same centre as that of the lower face of the intermediate plate, and which comprises a guide pin (16) which projects from said upper face (15) and which is intended to fit into said groove (11) so that it can pivot and slide across the latter,
said prosthesis being characterised in that
- the deepest point of the concave upper face (15) of the tibial element (5) is situated substantially forward of the geometric centre of said upper face;
- the guide pin (16) is situated substantially at said deepest point and comprises a body (17) of circular cross-section and a head (18), which is of greater width than the body (17) and which is also of circular cross-section; and
- the groove (11) disposed in the intermediate plate (8) has a cross-section which substantially corresponds to the profile of the guide pin (16), namely it is capable of receiving said pin (16) so that the latter is capable of pivoting and sliding but has a reduced capacity for vertical free motion.

2. A prosthesis according to claim 1, characterised in that the head (18) of the pin (16) is of substantially spherical shape.

3. A prosthesis according to claim 1 or claim 2, characterised in that the upper face of the intermediate plate (8), and therefore the concave surfaces which it comprises for receiving the rounded convex surfaces (4) of the femoral element (2) with the capacity of sliding, is inclined towards the back of the plate (8).

4. A prosthesis according to any one of claims 1 to 3, characterised in that the support plate (7) which forms the upper end of the tibial element (5) for receiving said intermediate plate (8) has a substantially constant thickness, namely it comprises a convex lower face (20) which has the shape of a portion of a spherical cap with the same centre as that of the upper face of said support plate (7).

5. A prosthesis according to any one of claims 1 to 4, characterised in that the anteroposterior groove (11) leads into the posterior side face of the intermediate plate (8).

6. A prosthesis according to any one of claims 1 to 4, characterised in that the groove (11) is closed at the posterior of the plate (8) by a transverse wall (21) forming a stop which limits the forward movement of the plate, said plate (8) comprising, substantially in the central part of the groove (11), two recesses (22) which permit the passage of the head (18) of the pin (16) with regard to the introduction of the latter into the groove (11).

## Patentansprüche

1. Kniegelenkprothese, umfassend:
- einerseits eine Zwischenplatte (8), die um eine vertikale Achse schwenkbar und in einer anteroposterioren Ebene schiebbar ist, die eine konvexe Unterseite (10) mit der Form eines Kugelkappenteils aufweist und die eine anteroposteriore, mediane Rille (11) umfaßt,
- andererseits ein tibiales Element (5), das eine konkave Oberseite (15) mit der Form eines Kugelkappenteils aufweist, der den gleichen Mittelpunkt wie die Unterseite der Zwischenplatte hat, und das einen Führungsteil (16) umfaßt, der von dieser Oberseite (15) vorspringt und dazu bestimmt ist, in die genannte Führungsrille (11) einzugreifen, wobei er in dieser geschwenkt und geschoben werden kann,
dadurch gekennzeichnet, daß
- die maximale Höhlung der konkaven Oberseite (15) des tibialen Elements (5) annähernd vor dem geometrischen Mittelpunkt dieser Oberseite angeordnet ist;
- der Führungsteil (16) annähernd auf der Höhe dieser maximalen Höhlung angeordnet ist und einen Körper (17) mit kreisförmigem Querschnitt und einen Kopf (18) mit größerer Breite als der Körper (17), ebenfalls mit kreisförmigem Querschnitt, umfaßt; und
- die Rille (11), die in der Zwischenplatte (8) gebildet ist, einen Querschnitt aufweist, der annähernd dem Profil des Führungsteils (16) entspricht, d.h. daß sie diesen Teil (16) mit der Möglichkeit zum Schwenken und Schieben, doch mit einer reduzierten Möglichkeit zu vertikalem Spiel, aufnehmen kann.

2. Prothese nach Anspruch 1, dadurch gekennzeichnet, daß der Kopf (18) des Teils (16) eine annähernd kugelförmige Form aufweist.

3. Prothese nach Anspruch 1 oder Anspruch 2, dadurch gekennzeichnet, daß die Oberseite der Zwischenplatte (8), und somit die konkaven Flächen, die sie umfaßt, um die abgerundeten konvexen Flächen (4) des femoralen Elements (2) mit der Möglichkeit zum Schieben aufzunehmen, in der Richtung der Hinterseite der Platte (8) geneigt ist.

4. Prothese nach einem der Ansprüche 1 bis 3, dadurch gekennzeichnet, daß die Stützplatte (7), die das obere Ende des tibialen Elements (5) bildet, um die genannte Zwischenplatte (8) aufzunehmen, eine annähernd konstante Dicke aufweist, d.h. daß sie eine konvexe Unterseite (20) umfaßt, die die Form eines Kugelkappenteils mit dem gleichen Mittelpunkt wie die Oberseite dieser Stützplatte (7) besitzt.

5. Prothese nach einem der Ansprüche 1 bis 4, dadurch gekennzeichnet, daß die anteroposteriore Rille (11) an der hinteren Seitenfläche der Zwischenplatte (8) mündet.

6. Prothese nach einem der Ansprüche 1 bis 4, dadurch gekennzeichnet, daß die Rille (11) im hinteren Bereich der Platte (8) durch eine Querwand (21) geschlossen wird, die einen Anschlag bildet, der die Bewegung der Platte nach vorne begrenzt, wobei die Platte (8) annähernd im mittleren Teil der Rille (11) zwei Einkerbungen (22) umfaßt, die die Passage des Kopfes (18) des Teils (16) im Hinblick auf seine Einführung in die Rille (11) ermöglichen.
